# EUROPEAN PATENT SPECIFICATION

(11) **EP 2 049 186 B1**
(45) Date of publication and mention of the grant of the patent: **16.10.2013**
(21) Application number: 07811072.3
(22) Date of filing: 02.08.2007
(51) Int. Cl.: A61M 25/10, A61M 29/02

(54) **CATHETER BALLOON WITH CONTROLLED FAILURE SHEATH**
KATHETERBALLON MIT SCHLEUSE MIT FEHLERKONTROLLE
BALLON DE CATHÉTER AVEC GAINE À DÉFAILLANCE CONTRÔLÉE

(30) Priority: 07.08.2006 US 501091
(43) Date of publication of application: 22.04.2009
(73) Proprietor: Gore Enterprise Holdings, Inc., Newark, DE 19714-9206 (US)
(72) Inventor: GREENE, Joel M., Flagstaff, AZ 86001 (US)
(74) Representative: Shanks, Andrew
(86) International application number: PCT/US2007/017372
(87) International publication number: WO 2008/021025

(56) References cited:
- EP-A- 1 595 569
- WO-A-01/80780
- WO-A-2005/018728
- WO-A-2006/107348
- US-A- 5 324 261
- US-B1- 6 355 013

## Description

### FIELD OF THE INVENTION

This invention relates generally to the field of balloons for balloon catheters, more particularly to such balloons incorporating controlled failure mechanisms, and methods of making such balloons.

### BACKGROUND

In transluminal angioplasty, a dilatation catheter having a balloon on the distal end is routed through the vascular system to the location of a stenotic lesion within a coronary artery. Following placement of the balloon across the lesion, a fluid is introduced into the proximal end of the catheter and is used to inflate the balloon to a predetermined relatively high pressure whereby the lesion is compressed into the vessel wall restoring patency to the previously occluded vessel. Catheter balloons can also be used to deliver and deploy balloon-expandable stents at the location of such lesions in order to maintain patency at the opened lesion.

In conventional stent-deploying balloon catheters, the balloon is typically made of essentially non-compliant material, such as nylon or polyethylene terephthalate (PET). Such non-compliant material exhibits little expansion in response to increasing levels of inflation pressure. Because the non-compliant material has a limited ability to expand, the uninflated balloon must be made sufficiently large that, when inflated, the balloon has sufficient working diameter to compress the stenosis and open the artery. However, a large profile non-compliant balloon can make the catheter difficult to advance through the patient's narrow vasculature because, in an uninflated condition, such balloons form flat or pancakeshaped "wings" which extend radially outward. Consequently, the wings of an uninflated balloon are typically all folded in the same circumferential direction to create a low profile configuration for introduction and advancement through the vessel. The wings are again produced upon deflation of the balloon following stent deployment within the patient. These wings on the deflated balloon are undesirable because they result in an increased balloon profile which can complicate withdrawing the catheter after stent deployment.

Expansion of such balloons that have been folded into a low profile configuration for introduction into the patient can cause non-uniform expansion of a stent mounted on the balloon. Likewise, non-uniform expansion can result in difficulty in using these previously folded balloons for opening stenotic lesions.

The non-uniform expansion of conventional designs has resulted in the use of an elastic sleeve around the balloon and under the stent to distribute force from the expanding folded balloon to the stent uniformly. Additionally, such an elastic sleeve can encourage the inflated balloon to deflate into a wingless compact profile. See, for example, U.S. Patent 5,116,318 to Hillstead.

Various controlled failure mechanisms are described previously for catheter balloons; see, for example, US Patent 6,375,637 to Campbell et al.

Catheter balloons that are reinforced with fibers are also known; see, for example, US Patent 4,706,670 to Andersen et al. While these fibers incorporated into the wall of a catheter balloon in addition to the overall material of the balloon wall results in a balloon having two dissimilar materials in its construction (i.e., two materials of different elastic modulus), the two materials do not provide a failure mechanism due to the uniformity of the two materials used in close proximity about the entire surface area of the balloon.

Typical non-compliant balloons have shown that at relatively high pressures, pinhole leaks may form which may create a high velocity jet of inflation fluid capable of damaging the adjacent blood vessel when it impinges on the vessel wall. Thus it is desirable for the balloon to be fabricated in such a way that it exhibits a controlled mode of failure, i.e., a rapid rupture so that the pressure is released over a larger area, thereby reducing the risk of damage to the adjacent vessel wall. However if the non-compliant balloon is surrounded by an elastic sheath, a pinhole leak or a rapid rupture of the underlying balloon causes the elastic sheath to rapidly expand in an unpredictable manner. This rapid, unpredictable expansion of the elastic sheath can potentially damage the vasculature, inhibit deflation and compromise the normal removal of the catheter.

WO 01/80780 describes a stent delivery system which includes a self dividing and retracting stent retaining sleeve having a ribbed configuration. The ribbed configuration provides the sleeve with a reduced columnar strength and improved radial strength characteristics. The stent delivery system further provides for a perforated portion on the sleeve which will rupture at a predetermined pressure thereby dividing the sleeve into a proximal portion and a distal portion. The ribbed sleeve portions provides a recoil action in opposite longitudinal directions to the individual sleeve portions which assists in actively retracting the sleeve portions off of the stent being expanded.

US 5,324,261 describes a balloon catheter which includes a sheath surrounding the balloon, the sheath having a longitudinal line of weakness and a drug-containing viscous matrix material intermediate between the balloon and the sheath such that when the balloon is positioned and inflated in the body lumen it causes the sheath to burst at the line of weakness and release viscous matrix material onto the lumen.

US 6,355,013 relates to a balloon catheter having a tubular basic body with distal and proximal ends, and at least one lumen extending between the proximal and the distal ends. A balloon is attached to the basic body close to the distal end which is connected with the lumen, so that the balloon may be selectively expanded by means of supplying a medium under pressure through the lumen. The balloon has at least one safety stop, which may extend between the distal and the proximal end of the balloon.

### SUMMARY OF THE INVENTION

The present invention is defined in attached claim 1.

The present invention is an improved elastic sheath designed to surround a conventional non-compliant angioplasty balloon. The improved sheath incorporates a failure control element that forces the sheath to fail concurrently with an underlying balloon. In particular the elastic sheath of the present invention ruptures rapidly and concurrently when the underlying balloon fails in an abrupt manner.

In a preferred embodiment, the elastic sheath includes an embedded, relatively inelastic component. Particularly preferred is an embedded, longitudinally oriented strip of relatively inelastic material having a different elastic modulus than that of the elastic sheath (e.g., a strip of ePTFE). The presence of the inelastic component provides a discontinuity in the compliant character of the elastic sheath, thereby providing a strain discontinuity in the sheath. This strain discontinuity allows the elastic sheath to fail by rupture (i.e., by tearing) over a significant length or area, thereby allowing the balloon pressure to be relieved over the larger area. This reduces the risk of damage to the vasculature as opposed to failures of balloon materials occurring over small areas (e.g., pinholes) that direct the relieved pressure to a very small area of the vasculature, resulting in significant damage to blood vessel walls.

### BRIEF DESCRIPTION OF THE DRAWINGS

- Figure 1: is a perspective view of a catheter assembly of the present invention, showing a distal balloon and elastic sheath portion including a controlled failure mechanism.
- Figure 2: is a partial longitudinal cross-sectional view of a catheter assembly, showing various portions of the catheter along with wall sections of a balloon and surrounding elastomeric sheath.
- Figure 3: is a partial side-view of a catheter assembly depicting a balloon and elastic sheath in an inflated state, wherein the elastic sheath includes a controlled failure mechanism integral to the wall of the sheath.
- Figure 4: is a partial side view of the balloon and elastic sheath of Figure 3 having failed by rupture.
- Figure 5A: is a transverse cross sectional view of an elastic sheath that includes an embedded inelastic strip.
- Figure 5B: is a transverse cross sectional view of an elastic sheath that includes a longitudinally oriented thinner notch of thickness engineered to fail at a predetermined pressure.
- Figure 6: is a process flow chart listing the general steps used in the fabrication of a controlled failure sheath according to the present invention.

### DETAILED DESCRIPTION OF THE DRAWINGS

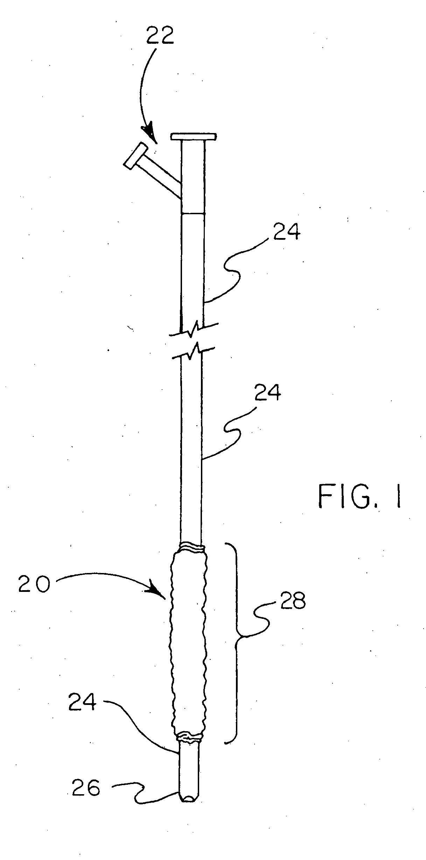

Figure 1 is a perspective view of a catheter assembly 20 including a proximal hub assembly 22 coupled to a catheter body 24. The catheter shaft 24 terminates at a distal catheter tip 26. Immediately proximal to the catheter distal tip 26 is a balloon portion 28.

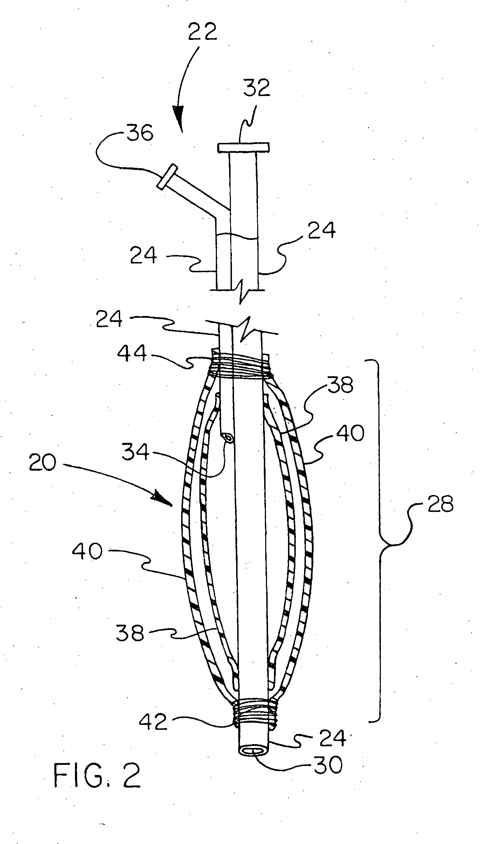

Figure 2 is a partial cross-sectional view of the catheter assembly 20 including a catheter 24 having a distal guidewire port 30. A guidewire lumen extends from the distal port 30 throughout the length of catheter 24 and terminates proximally at the hub port 32. Similarly, an inflation lumen has a distal port 34 terminating at the proximal hub port 36. Shown within the balloon portion 28, in longitudinal cross section for clarity of the underlying components, is balloon 38, surrounded by an elastic sheath 40. Elastic sheath 40 is shown attached to the distal end of catheter 24 by attachment means 42 such as an adhesive-coated wrapping of porous expanded polytetrafluoroethylene (hereinafter ePTFE) film 42. Attaching means may be provided in various ways known to those of skill in the art of balloon catheters, including adhesives, fiber wraps, film wraps, metal compression clamps, etc. Attaching means may also be radiopaque for convenient visualization of the location of the balloon within the vasculature. The proximal end of elastic sheath 40 is similarly attached to the catheter shaft 24 by attaching means 44.

The elastic sheath 40 has a relative high degree of longitudinal strength and has a relatively low degree of radial strength. The longitudinal strength prevents the folding of the sheath 40 during introduction into a hemostatic valve. The sheath 40 can be comprised of a suitable underlying ePTFE scaffold that is coated or imbibed with an elastomer. As an alternate, an elastomeric tube that incorporates high strength fibers, oriented along the tube longitudinal axis, may be used.

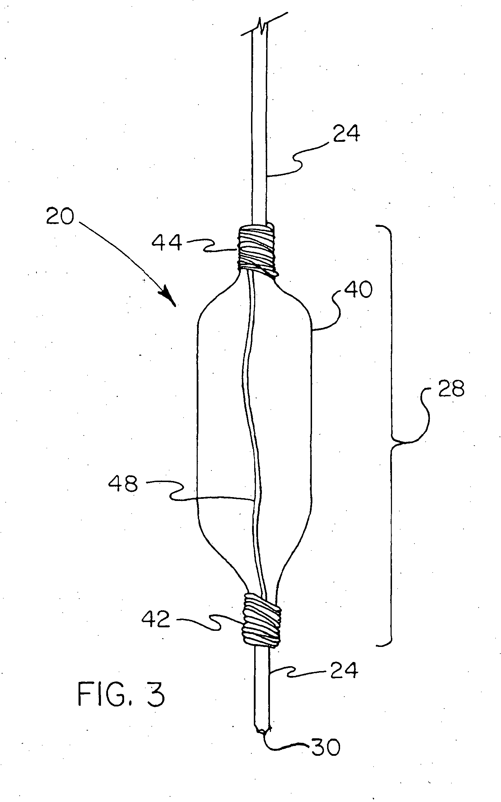

Figure 3 is a partial side-view of a catheter assembly 20 showing balloon portion 28 in an inflated state. Attached to, and more preferably embedded within elastomeric sheath 40, is a controlled failure mechanism in the form of a strip 48, as will be further described.

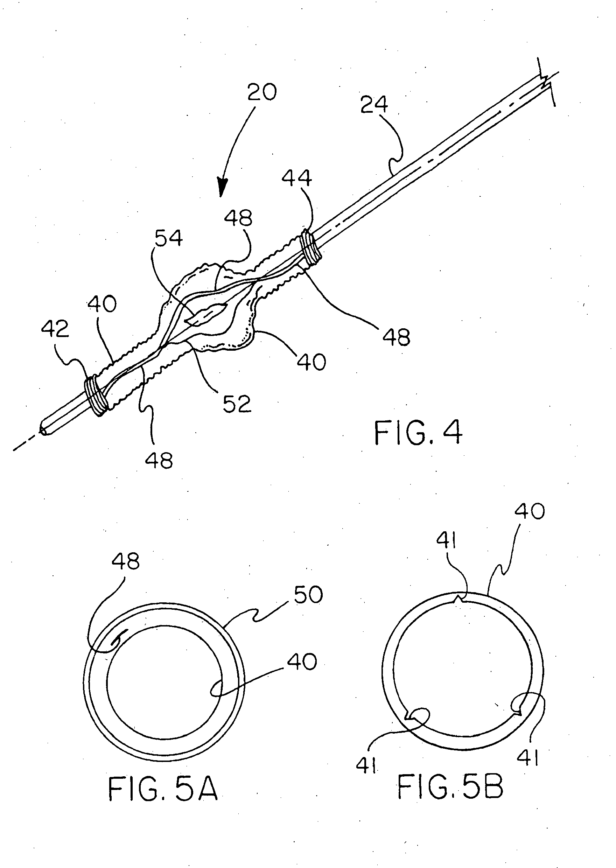

Figure 4 is a side view of a partial catheter assembly 20 including a catheter shaft 24 and attached elastic sheath 40 that has failed by rupture, indicated by the longitudinally oriented tear or rupture 52 through the wall of elastic sheath 40. The rupture 52 follows and is aligned to the controlled failure strip 48. Ruptured balloon 54 is visible through tear 52 in the wall of elastic sheath 40. When balloon 54 failed due to excessive inflation pressure, controlled failure of elastic sheath 40 resulting in the large tear 52 occurred immediately.

Figure 5A is a transverse cross sectional view of an elastic sheath 40 that includes an embedded inelastic strip 48. This embodiment is shown with the optional, preferred outer covering of an ePTFE tube 50.

As noted previously, the material of the inelastic embedded strip has a different elastic modulus from that of the elastic sheath. The difference may be 10%, 20%, 30%, 40%, 50%, 60%, 70%, 80%, 90%, 100% or more.

Alternate controlled failure mechanisms can include various "stress-risers" that are incorporated into the wall of the elastic sheath, for example notches or reliefs that are cut, extruded or otherwise formed into the sheath wall. Figure 5B is a transverse cross sectional view of an elastic sheath that includes one or more longitudinally oriented thinner notches 41 of thickness engineered to fail at a predetermined pressure. It is apparent that there are various ways to achieve similar failure mechanisms by including a weaker portion in the tubular elastic sheath 40.

To minimize the effect of a "pin-hole" leak in the underlying balloon, the elastomeric sheath can incorporate small diameter holes to allow the pressure to gradually bleed off.

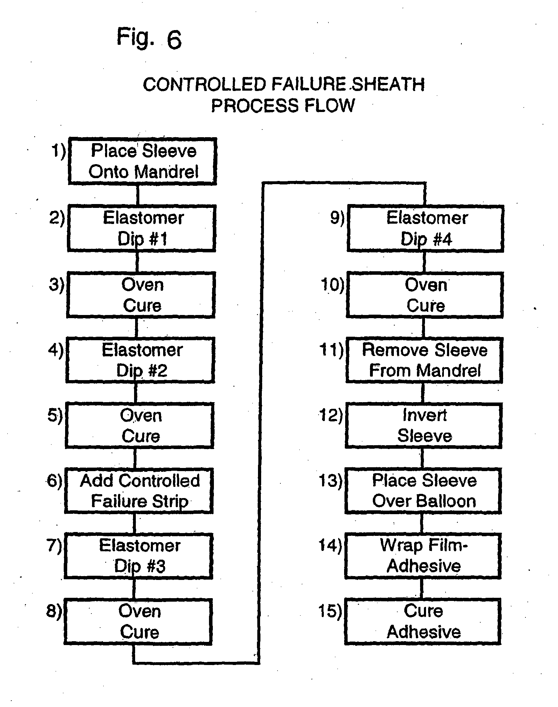

Figure 6 is a process flow chart listing the general steps used in the fabrication of a preferred controlled failure sheath. The following paragraphs detail each step:
1) A tube of ePTFE is placed over a mandrel. The longitudinally extruded and expanded tube has a relative high degree of longitudinal strength and has a relatively low degree of radial strength. Such a tube will therefore expand radially and resist longitudinal extension. The sleeve is preferably a tube with a thin wall, for example a wall less than 0.025mm. The tube wall thickness can range from about 0.008mm to about 0.38mm. Therefore the wall can have a thickness of about 0.008mm, about 0.010mm, about 0.013mm, about 0.015mm, about 0.018mm, about 0.020mm, about 0.023mm, about 0.025mm, about 0.028mm, about 0.0.030mm, about 0.033mm, about 0.035mm and about 0.038mm or greater. A preferred range of the tube wall thickness is from about 0.008mm to about 0.025mm, with a most preferred range of about 0.01mm to about 0.016mm.

The outer diameter of the tube can range from about 0.5mm to 10mm or greater. For example the tube can have an outer diameter of about 0.5mm, about 0.6mm, about 0.7mm, about 0.8mm, about 0.9mm, about 1.0mm, about 1.2mm, about 1.4mm, about 1,6mm, about 1.8mm, about 2mm, about 3mm, about 4mm, about 5mm, about 6mm, about 7mm, about 8mm, about 9mm and about 10mm or greater. The tube outer diameter has a preferred range of about 0.5mm to about 5mm, with a most preferred range of about 1 mm to about 1.5mm.

A typical tube length is about 25cm and can be any length compatible with the subsequent dipping process and desired balloon length. The mandrel is preferably slightly undersized to the tube inner diameter. For example, a tube with an outer diameter of about 1mm with a wall thickness of about 0.020mm will have an inner diameter of about 0.06mm. A slightly undersized mandrel will therefore have an outer diameter of about 0.05mm. After placing the tube onto the mandrel, the tube can be hand smoothed to remove any wrinkles. One end of the tube can protrude over one end of the mandrel and the overhanging tube end can be twisted to help secure the tube onto the mandrel.

An alternate embodiment of a tube that has a relative high degree of longitudinal strength and has a relatively low degree of radial strength is an elastomeric tube that incorporates high strength fibers that are oriented along the tube longitudinal axis.
2) The mandrel and surrounding tube are then dipped and imbibed into an elastomer solution. Any suitable elastomeric dispersion can be used. A typical suitable solution comprises BioSpan ® (segmented polyurethane, 24% ±2% solids content; from PTG Medical LLC, part number FP70001, Berkeley CA 94710), diluted with dimethylacetamide (DMAC; from Sigma-Aldrich, part number D5511, St. Louis MO). The elastomer solution is preheated to about 40-80°C in an appropriately sized test tube. The preheat temperature and preheat times as well as the solution solids content can be varied as required. The test tube with the preheated solution is then fixtured into a dipping fixture. The dipping fixture is comprised of a servo-driven, vertical cross-head with a clamping device suited to clamp and hold the mandrel in a vertical position (with the wrapped end of the tube pointing down). The servo-driven cross-head then descends and progressively submerges the mandrel and tube into the elastomer solution. A typical descent rate is about 0.1 to 2cm/second. About 15cm of the tube is submerged and held in the submerged position for a dwell time of about 10 to 90 seconds. The cross-head is then raised with an assent rate of about 0.1 to 2cm/second until the lower end of the mandrel and tube is removed from the elastomeric solution and test tube. Descent and assent rates as well as dwell times can be varied to control the amount of elastomer imbibed and coated onto the tube.
3) The mandrel with the dipped tube are then removed from the dipping fixture and placed into an air convection oven. The mandrel with the dipped tube is then pre-cured at about 50-80°C for about 1 to 3 minutes.
4) The mandrel and tube are then re-dipped and imbibed according to the previous step 2).
5) The mandrel and dipped tube are then pre-cured according to the previous step 3).
6) The controlled failure strip is then added to the exterior of the dipped tube. A preferred failure strip is pf ePTFE. While these may be created in a variety of ways, one method involves providing a second tube of ePTFE. According to previous step 1 this second ePTFE tube is flattened onto a cutting surface. The flattened tube is then cut longitudinally using a razor or any other suitable slitting means. By slitting the flattened tube with three, longitudinal and parallel cuts, four strips of material are formed, each strip having two layers of material and each strip having the same approximate width. The cut width of each strip can range between about 0.2 to about 0.5 of the tube outer diameter. For example for a tube having an outer diameter of about 1 mm, a slit tube controlled failure strip can have a width ranging from about 0.2mm to about 0.5mm. The mandrel and dipped tube from step 5) is then placed onto a compliant mat, for example a Berkshire UltraSeal 3000, part number US 3000.0909.8, from Berkshire Corp. MA. The compliant mat prevents densification of the ePTFE tube during the subsequent placement operation. The slit tube controlled failure strip is then positioned onto the exterior of the dipped tube. With slight hand tension, the controlled failure strip is oriented parallel to the tube longitudinal axis and is placed into contact with the dipped tube. The elastomer on the dipped tube has only been pre-cured and has a tacky surface, allowing retention of controlled failure strip onto the dipped tube.
7) The mandrel, dipped tube and attached controlled failure strip are then re-dipped and imbibed according to the previous step 2).
8) The mandrel, dipped tube and controlled failure strip are then pre-cured according to the previous step 3).
9) The mandrel, dipped tube and attached controlled failure strip are then re-dipped and imbibed according to the previous step 2).
10) The mandrel, dipped tube and controlled failure strip are then removed from the dipping fixture and placed into an air convection oven. The mandrel, dipped tube and controlled failure strip are then final-cured at about 50-80°C for about 45 to about 90 minutes. The resulting elastomeric tube forms a sheath that is radially expandable, resists longitudinal stretching and contains a controlled failure mechanism that is integral to the tube wall. The controlled failure mechanism is therefore in the form of a stress riser that is incorporated into the elastomeric sheath. Since the majority of the tube wall material is comprised of the elastomeric material (first material), the controlled failure mechanism is therefore comprised of a second material that is different than the first material.
11) The final cured tube/sheath from previous step 10) is then removed from the mandrel, and cut to a length that is approximately equal to the length of the desired balloon. A typical balloon length consists of a balloon "body", two opposing balloon "tapered ends" and two opposing balloon "legs." The overall cut length of the cured elastomeric sheath should be sufficient to cover the balloon body, both tapered ends and at least a portion of the two balloon legs.
12) The elastomeric sheath is then inverted. Since the elastomeric layers were built up on the external surface of the starting ePTFE thin walled tube, the inner surface of the sheath has a lubricious, primarily ePTFE surface. By inverting the sheath, the lubricious surface is now on the exterior of the sheath.
13) The inverted elastomeric sheath is then placed over a folded angioplasty balloon. A catheter having a folded and compacted non-distensible balloon is inserted into an introducer sheath. The conventional introducer sheath has on the proximal end a hemostatic valve and a flushing port/valve. The balloon can be advanced through the hemostatic valve and positioned within the introducer sheath near the distal end (of the sheath). One end of the inverted elastomeric sheath is then placed over the distal end of the introducer sheath. The elastomeric sheath can then be clamped and sealed onto the introducer sheath by the use of a conventional Tuohy-Borst compression fitting. The other end of the elastomeric sheath can then be closed and sealed by the use of a conventional hemostatic locking clamp. Once the elastomeric sheath is affixed and sealed to the introducer sheath, an inflating fluid can be injected into the introducer inflation port. Since the balloon catheter shaft is sealed by the hemostatic valve and the elastomeric sheath is closed and sealed to the introducer sheath, the inflating pressure will cause the elastomeric sheath to expand. After the elastomeric sheath is appropriately expanded, the balloon can be advanced and inserted into the expanded elastomeric sheath. The inflation pressure can then be bled-off, allowing the elastomeric sheath to deflate and retract down upon the compacted balloon.
14) To secure the sheath to the balloon legs, a film wrapping and a UV curable adhesive is applied to the ends of the elastomeric sheath. An ePTFE or any other suitable thin film is wrapped under tension onto the ends of the elastomeric sheath. The film wrapping compresses the elastomeric sheath down onto the balloon leg portions. A suitable UV curable adhesive is then applied onto the wrapped film.
15) The covered balloon is then placed under a UV source to cure the adhesive, resulting in a non-compliant balloon covered by a controlled failure elastomeric sheath as generally shown in Figures 1, 2 and 3.

### EXAMPLE

A preferred embodiment of a controlled failure elastomeric sheath covering a non-elastic balloon is detailed in Example 1. This example follows the process flow outlined according to Figure 5.
1) A tube of ePTFE was placed over a mandrel. The longitudinally extruded and expanded tube had a relative high degree of longitudinal strength and had a relatively low degree of radial strength. The tube therefore expanded radially and resisted longitudinal extension. The ePTFE tube was extruded and expanded by stretching in the direction of the longitudinal axis of the tube; it had a wall thickness of about 0.013mm, an outer diameter of about 1.35mm, a mean fibril length of about 30 micrometers and was about 25cm long. This tube was placed over a mandrel having an outer diameter of about 1.34mm. After the tube was placed onto the mandrel, the tube was hand smoothed to remove any wrinkles. One end of the tube protruded over one end of the mandrel and the overhanging tube end was twisted to help secure the tube onto the mandrel.
2) The mandrel and surrounding tube was then dipped and imbibed into an elastomer solution. A solution of BioSpan® (segmented polyurethane, 24% ±2% solids content; from PTG Medical LLC, part number FP70001, Berkeley CA 94710) which is diluted to about 12% (weight percent) solids content using dimethylacetamide (DMAC; from Sigma-Aldrich, part number D5511, St. Louis MO) to about 12% (weight percent) solids content. This solution was preheated to about 60°C in 20cm long by 1.3cm diameter test tube. The test tube with the preheated solution was then fixtured into a dipping fixture. The dipping fixture was comprised of a servo-driven, vertical cross-head with a clamping device suited to clamp and hold the mandrel in a vertical position (with the wrapped end of the tube pointing down). The servo-driven cross-head then descended and progressively submerged the mandrel and tube into the elastomer solution. The descent rate was about 0.6cm/second. About 15cm of the tube was submerged and held in the submerged position for a dwell time of about 30seconds. The cross-head was then raised with an assent rate of about 0.3cm/second until the lower end of the mandrel and tube was removed from the elastomeric solution and test tube.
3) The mandrel with the dipped tube was then removed from the dipping fixture and placed into an air convection oven. The mandrel with the dipped tube was then pre-cured by being placed into an air convection oven set at about 65°C for about 2 minutes.
4) The mandrel and tube were then re-dipped and imbibed according to the previous step 2).
5) The mandrel and dipped tube were then pre-cured according to the previous step 3).
6) The controlled failure strip was then added to the exterior of the dipped tube. A second tube of ePTFE, according to previous step 1 was flattened onto a cutting surface. The flattened tube was then cut longitudinally using a razor. By slitting the flattened tube with three, longitudinal and parallel cuts, four strips of material were formed, each strip having two layers of material and each strip having the same approximate width of about 0.5mm. The mandrel and dipped tube from step 5) was then placed onto a compliant mat, for example a Berkshire UltraSeal 3000, part number US 3000.0909.8, from Berkshire Corp. MA. The compliant mat prevented densification of the ePTFE tube during the subsequent placement operation. The slit tube controlled failure strip was then positioned onto the exterior of the dipped tube. With slight hand tension, the controlled failure strip was oriented parallel to the tube longitudinal axis and was placed into contact with the dipped tube. The elastomer on the dipped tube has only been pre-cured and had a tacky surface, allowing retention of controlled failure strip onto the dipped tube.
7) The mandrel, dipped tube and attached controlled failure strip were then re-dipped and imbibed according to the previous step 2).
8) The mandrel, dipped tube and controlled failure strip were then pre-cured according to the previous step 3).
9) The mandrel, dipped tube and attached controlled failure strip were then re-dipped and imbibed according to the previous step 2).
10) The mandrel, dipped tube and controlled failure strip were then removed from the dipping fixture and placed into an air convection oven. The mandrel, dipped tube and controlled failure strip were then final-cured at about 65°C for about 1 hour. The resulting elastomeric tube formed a sheath that was radially expandable, resisted longitudinal stretching and contained a controlled failure mechanism that, while being of a different material than the remainder of the tube wall, was incorporated into the tube wall.
11) The final cured tube/sheath from previous step 10) was then removed from the mandrel, and cut to a length that was approximately equal to the length of the desired balloon. A typical balloon length consists of a balloon "body", two opposing balloon "tapered ends" and two opposing balloon "legs." The overall cut length of the cured elastomeric sheath should be sufficient to cover the balloon body, both tapered ends and at least a portion of the two balloon legs. The elastomeric sheath was cut to a length of about 90mm. This cut length was suitable to cover a balloon having a length (body, tapered ends and leg portions) of about 90mm.
12) The elastomeric sheath was then inverted. Since the elastomeric layers were built up on the external surface of the starting ePTFE thin walled tube, the inner surface of the sheath had a lubricious, primarily ePTFE surface. By inverting the sheath, the lubricious surface was now on the exterior of the sheath.
13) The inverted elastomeric sheath was then placed over a folded angioplasty balloon. A balloon catheter, having a balloon of PET with an inflated body diameter of about 8mm, a body length of about 40mm, shoulder lengths of about 18mm (20° included angle), leg lengths of about 7mm and leg diameters of about 1.4mm (from Advanced Polymers Inc., NH), was procured. The catheter and the folded non-distensible balloon were then inserted into a 7Fr introducer sheath (Avanti®, 7F, 11 cm, part number 504-607X, from Cordis Corp., Miami Lakes FL). This conventional introducer sheath had on the proximal end a hemostatic valve and a flushing port/valve. The balloon was advanced through the hemostatic valve and positioned within the introducer sheath near the distal end (of the sheath). One end of the inverted elastomeric sheath was then placed over the distal end of the introducer sheath. The elastomeric sheath was then be clamped and sealed onto the introducer sheath by the use of a conventional Tuohy-Borst compression fitting (Large Diameter Part # 11183, from Qosina, Edgewood NY). The other end of the elastomeric sheath was then be closed and sealed by the use of a conventional hemostatic locking clamp. Once the elastomeric sheath was affixed and sealed to the introducer sheath, a water inflating fluid was injected into the introducer inflation port. Since the balloon catheter shaft was sealed by the hemostatic valve and the elastomeric sheath was closed and sealed to the introducer sheath, the inflating pressure caused the elastomeric sheath to expand. After the elastomeric sheath was appropriately expanded, the balloon was advanced and inserted into the expanded elastomeric sheath. The inflation pressure was then bled-off, allowing the elastomeric sheath to deflate and retract down upon the compacted balloon.
14) To secure the sheath to the balloon legs, a film wrapping and a UV curable adhesive is applied to the ends of the elastomeric sheath. An ePTFE film about 2mm wide by about 0.005mm thick, having an approximate mean fibril length of about 50 micrometers, was wrapped under tension onto the ends of the elastomeric sheath. The film wrapping compressed the elastomeric sheath down onto the balloon leg portions. A UV curable adhesive (Grade 3381 from Loctite Corp., Rocky Hill CT) was then applied onto the wrapped film.
15) The covered balloon was the placed under a UV source to cure the adhesive, resulting in a non-compliant balloon covered by a controlled failure elastomeric sheath as generally shown in Figures 1, 2 and 3.

The covered balloon from step 15) above was the burst tested. The balloon was progressively inflated through the catheter inflation port, using an Interface and Associates Tester, Model PT3070, Laguna Niguel CA. The inflation pressures were progressively increased and recorded until the underlying balloon abruptly burst at a pressure of about 22atm. The inflation rate for the balloon was about 0.1ml/sec. The elastomeric sheath also abruptly failed, concurrently with the balloon failure. The burst balloon and cover are described by Figure 4.

## Claims

1. A balloon catheter (20), comprising:
non-compliant balloon (38) surrounded by an external elastomeric sheath (40);
said elastomeric sheath (40) having a controlled failure mechanism (48) whereby the sheath (40) ruptures concurrently with failure of the balloon (38) by rupture, wherein the rupture of the sheath (40) occurs over a larger area than does the rupture of the balloon (38).

2. The balloon catheter of claim 1 wherein the controlled failure mechanism comprises a stress riser (41) incorporated into the elastomeric sheath (40).

3. The balloon catheter of claim 2 wherein the stress riser is formed by a longitudinal notch (41) in a wall of the elastomeric sheath (40).

4. The balloon catheter of claim 2 wherein the stress riser is formed by a circumferential notch in a wall of the elastomeric sheath.

5. The balloon catheter of claim 2 wherein the elastic sheath (40) is substantially comprised of a first material; and
the stress riser is formed by a sheath wall portion (48)having a second material that is different than the first material.

6. The balloon catheter of claim 2 wherein the stress riser is formed by heat treating selective wall portions of the elastomeric sheath.

7. The balloon catheter of claim 2 wherein the stress riser is formed by work hardening selective wall portions of the elastomeric sheath.

8. The balloon catheter of claim 2 wherein the elastic sheath is substantially comprised of a first material;
the stress riser formed by a ribbon of a second material different than the first material; and
the ribbon is embedded into the elastomeric sheath.

9. The balloon catheter of claim 2 wherein the elastic sheath is substantially comprised of a first material;
the stress riser formed by a ribbon of a second material different than the first material; and
the ribbon is attached to a surface of the elastomeric sheath.

10. The balloon catheter of claim 1 wherein the elastomeric sheath (40) concentrically expands to an inflated state.

11. The balloon catheter of claim 1 wherein the elastomeric sheath (40) efficiently compacts to a deflated state.

12. The balloon catheter of claim 1 wherein the elastomeric sheath (40) concentrically expands to an inflated state and efficiently compacts to a deflated state.

13. The balloon catheter of claim 1, wherein the non-compliant balloon (38) has two opposing ends; and
the elastomeric sheath (40) is attached to the two opposing ends of the non-compliant balloon (38).

## Patentansprüche

1. Ballonkatheter (20), der aufweist:
einen nicht nachgiebigen Ballon (38), der durch eine äußere elastomere Schleuse (40) umgeben wird;
wobei die elastomere Schleuse (40) einen Mechanismus (48) mit Fehlerkontrolle aufweist, wodurch die Schleuse (40) gleichzeitig mit dem Versagen des Ballons (38) durch einen Bruch zerreißt, wobei der Bruch der Schleuse (40) über einen größeren Bereich auftritt als der Bruch des Ballons (38).

2. Ballonkatheter nach Anspruch 1, bei dem der Mechanismus mit Fehlerkontrolle eine Belastungszone (Stress riser) (41) aufweist, die in der elastomeren Schleuse (40) eingebaut ist.

3. Ballonkatheter nach Anspruch 2, bei dem die Belastungszone durch eine Längskerbe (41) in einer Wand der elastomeren Schleuse (40) gebildet wird.

4. Ballonkatheter nach Anspruch 2, bei dem die Belastungszone durch eine Umfangskerbe in einer Wand der elastomeren Schleuse (40) gebildet wird.

5. Ballonkatheter nach Anspruch 2, bei dem die elastische Schleuse (40) im Wesentlichen ein erstes Material aufweist; und
die Belastungszone durch einen Schleusenwandabschnitt (48) gebildet wird, der ein zweites Material aufweist, das vom ersten Material abweichend ist.

6. Ballonkatheter nach Anspruch 2, bei dem die Belastungszone durch Wärmebehandlung von selektiven Wandabschnitten der elastomeren Schleuse gebildet wird.

7. Ballonkatheter nach Anspruch 2, bei dem die Belastungszone durch Kaltverfestigung von selektiven Wandabschnitten der elastomeren Schleuse gebildet wird.

8. Ballonkatheter nach Anspruch 2, bei dem die elastische Schleuse im Wesentlichen ein erstes Material aufweist;
die Belastungszone durch ein Band aus einem zweiten Material gebildet wird, das vom ersten Material abweichend ist; und
das Band in die elastomere Schleuse eingebettet ist.

9. Ballonkatheter nach Anspruch 2, bei dem die elastische Schleuse im Wesentlichen ein erstes Material aufweist;
die Belastungszone durch ein Band aus einem zweiten Material gebildet wird, das vom ersten Material abweichend ist; und
das Band an einer Fläche der elastomeren Schleuse befestigt ist.

10. Ballonkatheter nach Anspruch 1, bei dem sich die elastomere Schleuse (40) konzentrisch zu einem aufgeblasenen Zustand ausdehnt.

11. Ballonkatheter nach Anspruch 1, bei dem sich die elastomere Schleuse (40) wirksam zu einem entleerten Zustand verdichtet.

12. Ballonkatheter nach Anspruch 1, bei dem sich die elastomere Schleuse (40) konzentrisch zu einem aufgeblasenen Zustand ausdehnt und wirksam zu einem entleerten Zustand verdichtet.

13. Ballonkatheter nach Anspruch 1, bei dem der nicht nachgiebige Ballon (38) zwei entgegengesetzte Enden aufweist; und
die elastomere Schleuse (40) an den zwei entgegengesetzten Enden des nicht nachgiebigen Ballons (38) befestigt ist.

## Revendications

1. Cathéter à ballonnet (20), comprenant :
un ballonnet non élastique (38), entouré par une gaine élastomère externe (40) ;
ladite gaine élastomère (40) comportant un mécanisme à défaillance contrôlée (48), la rupture de la gaine (40) étant ainsi simultanée à la défaillance du ballonnet (38) par rupture, la rupture de la gaine (40) se faisant au-delà d'une zone plus grande que la rupture du ballonnet (38).

2. Cathéter à ballonnet selon la revendication 1, dans lequel le mécanisme à défaillance contrôlée comprend un élément d'accroissement de la contrainte (41) incorporé dans la gaine élastomère (40).

3. Cathéter à ballonnet selon la revendication 2, dans lequel l'élément d'accroissement de la contrainte est formé par une encoche longitudinale (41) dans une paroi de la gaine élastomère (40).

4. Cathéter à ballonnet selon la revendication 2, dans lequel l'élément d'accroissement de la contrainte est formé par une encoche circonférentielle dans une paroi de la gaine élastomère.

5. Cathéter à ballonnet selon la revendication 2, dans lequel la gaine élastomère (40) est pour l'essentiel composée d'un premier matériau ; et
l'élément d'accroissement de la contrainte étant formé par une partie de paroi de la gaine (4B) comportant un deuxième matériau différent du premier matériau.

6. Cathéter à ballonnet selon la revendication 2, dans lequel l'élément d'accroissement de la contrainte est formé par traitement thermique de parties de paroi sélectives de la gaine élastomère.

7. Cathéter à ballonnet selon la revendication 2, dans lequel l'élément d'accroissement de la contrainte est formé durcissement à froid de parties de paroi sélectives de la gaine élastomère.

8. Cathéter à ballonnet selon la revendication 2, dans lequel la gaine élastomère est pour l'essentiel composée d'un premier matériau ;
l'élément d'accroissement de la contrainte étant formé par un ruban composé d'un deuxième matériau différent du premier matériau ; et
le ruban étant noyé dans la gaine élastomère.

9. Cathéter à ballonnet selon la revendication 2, dans lequel la gaine élastomère est pour l'essentiel composée d'un premier matériau ;
l'élément d'accroissement de la contrainte étant formé par un ruban composé d'un deuxième matériau différent du premier matériau ; et
le ruban étant fixé sur une surface de la gaine élastomère.

10. Cathéter à ballonnet selon la revendication 1, dans lequel la gaine élastomère (40) est dilatée de manière concentrique vers un état gonflé.

11. Cathéter à ballonnet selon la revendication 1, dans lequel la gaine élastomère (40) est compactée de manière efficace vers un état dégonflé.

12. Cathéter à ballonnet selon la revendication 1, dans lequel la gaine élastomère (40) est dilatée de manière concentrique vers un état gonflé et compactée de manière efficace vers un état dégonflé.

13. Cathéter à ballonnet selon la revendication 1, dans lequel le ballonnet non élastique (38) comporte deux extrémités opposées ; et
la gaine élastomère (40) étant fixée sur les deux extrémités opposées du ballonnet non élastique (38).
